# EUROPÄISCHE PATENTANMELDUNG

(11) **EP 2 476 455 A1**
(43) Veröffentlichungstag der Anmeldung: **18.07.2012**
(21) Anmeldenummer: 11194024.3
(22) Anmeldetag: 16.12.2011
(51) Int. Cl.: A61N 1/05, A61M 25/00

(54) **Implantierbare Elektrodenleitung**

(30) Priorität: 13.01.2011 US 201161432212 P
(71) Anmelder: BIOTRONIK SE & Co. KG, 12359 Berlin (DE)
(72) Erfinder: Kolberg, Gernot, 12161 Berlin (DE); Palm, Jochen, 15831 Mahlow (DE); Bartels, Klaus, 10115 Berlin (DE)
(74) Vertreter: Lindner-Vogt, Karin L.

(57) **Zusammenfassung**

Implantierbare Elektrodenleitung zur Übertragung elektrischer Impulse an reizbares Körpergewebe und/oder von an Körpergewebe abgegriffenen elektrischen Signalen zu einer Erfassungseinheit, mit einer distalen Elektrode, einem proximalen Elektrodenanschluss und einer die oder jede Elektrode mit dem oder jedem Elektrodenanschluss verbindenden, in einem Leitungskörper verlaufenden Elektrodenzuleitung, wobei der Leitungskörper eine gelenkige Reihung von harten Gliedern aufweist.

## Beschreibung

Die Erfindung betrifft eine implantierbare Elektrodenleitung zur Übertragung elektrischer Impulse an reizbares Körpergewebe und/oder von an Körpergewebe abgegriffenen elektrischen Signalen zu einer Erfassungseinheit, mit einer distalen Elektrode, einem proximalen Elektrodenanschluss und einer die oder jede Elektrode mit dem oder jedem Elektrodenanschluss verbindende, oder zur Übertragung von Elektroschocks oder zur Ansteuerung von Sensoren dienende, in einem Leitungskörper verlaufende Elektrodenleitung.

Derartige Elektrodenleitungen, mit denen etwa Stimulationsimpulse von Herzschrittmachern an das Herz und ggf. auch am Herzen auftretende Aktionspotentiale zum Schrittmacher oder die Schockimpulse eines implantierten Kardioverters an das Herz und ggf. auch hier abgefühlte Aktionspotentiale zum Kardioverter übertragen werden oder mit dem auch Hirnbereiche oder Nerven stimuliert oder dort abgefühlte elektrische Signale an ein Erfassungs- und Auswertungsgerät übertragen werden, sind massenhaft im klinischen Einsatz.

Von den zahlreichen Einsatzfeldern der Elektrodenleitungen gibt es einige, bei denen sie zumindest in Teilabschnitten hohen mechanischen Belastungen ausgesetzt sind, die im Langzeiteinsatz die Funktionsfähigkeit beeinträchtigen oder die Elektrodenleitung sogar vollständig außer Funktion setzen können. Beispiele hierfür sind Schrittmacherelektrodenleitungen, eine oder mehrere Zuleitungen zwischen einem implantierten Steuergerät und einem oder mehrerer implantierbarer Sensoren, oder ICD-Elektroden, die eine oder mehrere sehr große Flächen zum großflächigen Applizieren sehr hoher Stromimpulse in das Gewebe aufweisen.

Zunächst wird überschüssige Elektrodenlänge in der Schrittmachertasche eingeschlossen. Das Gebilde wird mit einer zähen Bindegewebshaut umwachsen. An den Stellen, an denen die Elektrode das Gehäuse berührt oder sich mit anderen Elektrodenabschnitten kreuzt, kann es zu hohen Druckbelastungen auf den Leitungskörper kommen, da das umwachsende Bindegewebe die Elektrode nicht ausweichen lässt. Im Anschluss daran passiert die Elektrode den Bereich zwischen Schlüsselbein und 1. Rippenbogen. Hier kann es bei ungünstiger Lage der Elektrode zu einem Quetschen kommen.

In der Vergangenheit hat man im Rahmen umfangreicher Entwicklungen verschiedene Lösungsansätze für dieses Problem bereitgestellt. Elektrodenleitungen sind hoch flexibel gestaltet. Die verwendeten harten Werkstoffe wie Metall für die Zuleitungen sind so gestaltet, dass ihre Konstruktion sehr flexibel ist. Drähte werden zu Wendeln gewickelt oder sehr dünn zu Seilen verflochten. Als Isolatoren werden weiche und möglichst elastische Kunststoffe eingesetzt, die den Bewegungen der Elektrode möglichst wenig Widerstand bieten.

Die bekannten Lösungen konnten bisher in der Praxis nicht vollständig überzeugen. Bei radialem Druck weicht das Isolationsmaterial einerseits aus, so dass der Druck letztlich auf die Zuleitungen wirkt. Andererseits wird durch die Quetschung des Isolationsmaterials der Kunststoff gestresst. Der Stress kann zu einer Degradation des Materials oder direkt zu einem mechanischen Nachgeben führen. Es kommt zum Abreiben, Aufplatzen oder Abbau der Isolation. Zunächst entsteht eine Verletzung der Isolation. Es kann Körperflüssigkeit in die Elektrode eindringen, die zwischen den Leitern Elektrolytbrücken schließen kann. Durch Neben- oder Kurzschlüsse kann die Therapie beeinträchtigt werden. Im schlimmsten Fall aber kommt es zum Bruch der Zuleitungen und zum Ausfall der Therapie. Es kann außerdem nicht ausgeschlossen werden, dass ein gebrochener Elektrodenkörper weiteren Schaden anrichtet.

Der Erfindung liegt daher die Aufgabe zugrunde, eine verbesserte Elektrodenleitung bereit zu stellen, die insbesondere zumindest in bestimmten Abschnitten widerstandsfähiger gegenüber im Wesentlichen radial einwirkenden Kräften und Reibung ist, jedoch weiterhin die erforderliche Flexibilität aufweist.

Diese Aufgabe wird durch eine Elektrodenleitung mit den Merkmalen des Anspruchs 1 gelöst. Zweckmäßige Fortbildung des Erfindungsgedankens ist Gegenstand der abhängigen Ansprüche.

Unter dem Begriff "harte Glieder" werden hier separate Elemente oder auch abgrenzbare Abschnitte im Längsverlauf eines Leitungskörpers verstanden, die hochgradig widerstandsfähig ("hart") gegenüber radial oder schräg zur Längsachse der Elektrodenleitung einwirkenden Kräften und relativ kurz gegenüber der Gesamtlänge der Elektrodenleitung sind. Mit der Erfindung wird vorgeschlagen, zumindest solche Abschnitte im Längsverlauf einer Elektrodenleitung, die typischerweise stark derartigen mechanischen Belastungen ausgesetzt sind, besonders widerstandsfähig auszugestalten.

Eine Elektrodenleitung, die nach der erfindungsgemäßen Lösung aufgebaut ist, ist wesentlich stabiler gegenüber den mechanischen Belastungen, denen sie im praktischen Einsatz ausgesetzt ist. Die einwirkenden radialen Druck- und Walkkräfte werden hier von einem zusätzlichen Schild, den harten Gliedern, aufgenommen. Die Funktionskomponente Zuleitung, bestehend aus Seil und/oder Wendel, sowie die Isolatoren, bestehend aus Kunststoff, werden auf ihre eigentliche Funktion beschränkt (Leiten bzw. Isolieren). Durch die auf sie einwirkenden radialen Kräfte mussten diese Funktionselemente bei herkömmlichen Elektroden verschiedenen Beanspruchungen wie Torsionsmomenten, Zug-, Walkkräften und Reibung, widerstehen. Eine optimale Auslegung der erfindungsgemäßen Lösung ermöglicht zudem einen dauerhaften Schutz gegen ungewollte Bewegungen des Elektrodenkörpers. So können Relativbewegungen zwischen Zuleitung und Isolation minimiert werden

Wesentliche Aspekte von Ausführungsformen der Erfindung sind die Folgenden:
1. Werkstoffe für mindestens einen Teil der harten Glieder sind:
   Metall: Platin, Tantal, Iridium, Palladium, Stahl, MP35N, Gold, .., Keramik: A1203, Zr02, Ti02, MgO, ZnO, Aluminiumtitanat (A1203 + Ti02), Bariumtitanat (BaO + Ti02), Siliciumcarbid (SiC), Berylliumoxid (BeO), Aluiniumnitrid (AlN), Hafniumcarbid (HfC), Tantalcarbid (TaC), Titannitrid (TiN), Bornitrid (BN), Borcarbid (B4C), Wolframcarbid (WC), Siliciumnitrid (Si3N4),
      Glas
      Kunststoff: PEEK, Silikon, div. Copolymere, Polyimid, PA, hochdichtes Polyethylen, Polysulfon, oder faser- oder nanopartikel gefüllten Varianten der genannten Kunststoffe
2. Die harten Elemente (Glieder) wechseln sich mit elastischen Elementen (Abschnitten) ab.
3. Die Beschaffenheit der Elemente verändert sich über den Verlauf der Elektrode.
4. Die harten Glieder der Reihung ("Kette") sind miteinander durch einen elastischen Werkstoff verbunden.
5. Der elastische Werkstoff wird durch Extrusion oder Beschichtung oder Umspritzen der Kette aufgebracht.
6. Die Glieder der Kette sind von einem elastischen Werkstoff umhüllt.
7. Der Zuleitungskörper wird mit einem abriebfesten Schlauch umgeben.
8. Im Kern der Kette verläuft beispielsweise in einem Lumen mindestens eine Wendel oder ein Versteifungsdraht
9. Im Kern der Kette verläuft mindestens ein Seil.
10. Die Wendel oder das Seil/die Seile sind voneinander und/oder gegenüber der Kette isoliert.
11. Die Freimachungen sind unsymmetrisch (es muss kein Kernlumen geben).
12. Die Elemente der Kette sind Isolatoren.
13. Die Elemente der Kette sind Halbleiter.
14. Die Elemente der Kette sind leitfähig.
15. Die Form der Elemente wechselt sich je nach Funktion ab.
16. Einzelne Glieder haben unterschiedliche Länge.
17. Einzelne Glieder haben unterschiedlichen Durchmesser.
18. Einzelne Glieder der Kette sind als Ringelektrode gestaltet oder können eine Ringelektrode aufnehmen.
19. Einzelne Glieder der Kette sind als Sensoren gestaltet oder können Sensoren aufnehmen.
20. Einzelne Glieder der Kette sind als Spulen ausgelegt oder können Spulen aufnehmen.
21. Einzelne Glieder der Kette sind als Kondensatoren ausgelegt oder können Kondensatoren aufnehmen.
22. Einzelne Glieder der Kette enthalten elektronische Bauelemente, analoge und/oder digitale Schaltkreise, Akkumulatoren, Batterien, Antennen, Sender und/oder Empfänger.
23. Einzelne Glieder der Kette sind als Fixationselement ausgelegt oder können Fixationselemente aufnehmen, mit der die Elektrode an ihrem Bestimmungsort fixiert wird.
24. Die Glieder haben für die außermittig verlaufenden Zuleitungen Freimachungen, die die Bahn vorgeben, in denen sie geführt werden.
25. Die außermittig verlaufenden Zuleitungen sind parallel zur Achse des Elektrodenkörpers angeordnet.
26. Die außermittig verlaufenden Zuleitungen sind gewendelt zur Achse des Elektrodenkörpers angeordnet.
27. Die außermittig verlaufenden Zuleitungen sind gewendelt zur Achse des Elektrodenkörpers angeordnet, wobei die Steigung der Wendel über die Elektrodenlänge wechselt, sich umkehrt (entgegengesetzter Wickelsinn) oder gegen unendlich geht, d.h. parallel verläuft.
28. Die Stirnseiten (Kontaktflächen) der Glieder zu den benachbarten Gliedern sind so gestaltet (abgeflacht), dass eine Biegung der Kette erleichtert wird.
29. Die Kontaktflächen der Glieder sind so gestaltet, dass bei einer Biegung der minimale Biegeradius der Kette eingeschränkt wird.
30. Die Elemente sind so gestaltet, dass der Freiheitsgrad der Bewegung zu den benachbarten Gliedern eingeschränkt ist. Glieder übernehmen Gelenkfunktion (d.h. die Kette kann sich an diesem Übergang der Kettenglieder nicht mehr in alle Richtungen biegen), wobei die als Gelenke ausgelegten Glieder so gestaltet sind, dass sie Zugkräfte aufnehmen können (eine so genannte "Hinterkrallung").
31. Die Bewegungsebene zum nachfolgenden Glied ist um einen Winkel, z.B. 90°, verdreht.
32. Die außermittig verlaufenden Zuleitungen werden in der Gelenkebene von einem Glied zum nächsten geführt (so wird Relativbewegung der Zuleitung zum Glied minimiert).
33. Die Glieder werden auf einen Schlauch angespritzt oder aufextrudiert.
34. Die oben aufgeführten Glieder werden durch spezielle Glieder voneinander getrennt, die die Funktion von Gelenken übernehmen.
35. Die Glieder sind durch integrierte Gelenke miteinander verbunden.
36. Die "Glieder" sind aus einem Rohr gefertigt.
37. Die Glieder sind schuppenartig überlappend aneinander gereiht.
38. Die Abschnitte des Rohres sind miteinander verbunden.
39. Das Rohr ist, insbesondere mittels Laserstrahlschnitt, aus einem zusammenhängenden Stück gefertigt.
40. Die Art der Kette wechselt über den Verlauf des Elektrodenkörpers. Segmente des Elektrodenkörpers werden als Kette ausgelegt, andere verwenden herkömmliche Konstruktionsprinzipien des Elektrodenkörpers.

Vorteile und Zweckmäßigkeiten der Erfindung ergeben sich im Übrigen aus den nachfolgenden Beschreibungsbeispielen anhand der Figuren. Von diesen zeigen:
- Fig. 1: eine schematische Darstellung einer herkömmlichen implantierbaren Elektrodenleitung,
- Fig. 2: in einer perspektivischen Schnittsdarstellung ein Beispiel einer hochentwickelten Elektrodenleitung mit mehreren in einem Leitungskörper aufgenommenen Zuleitungen,
- Fig. 3: in einer perspektivischen Schnittdarstellung eine weitere hochentwickelte Elektrodenleitung mit mehreren Zuleitungen in koaxialer Anordnung,
- Fig. 4A-4B und 5A-5B: jeweils schematische Darstellungen einer erfindungsgemäß ausgebildeten Elektrodenleitung in Seitenansicht,
- Fig. 6A bis 6C: in schematischen perspektivischen Schnittansichten jeweils ein hartes Glied einer Ausführungsform der erfindungsgemäßen Elektrodenleitung,
- Fig. 7: eine schematische Seitenansicht eines Abschnitts einer weiteren erfindungsgemäßen Elektrodenleitung,
- Fig. 8: perspektivische Darstellungen von drei harten Gliedern einer Ausgestaltung der in Fig. 7 gezeigten Elektrodenleitung,
- Fig. 9: eine perspektivische Darstellung von einander benachbarten harten Gliedern einer weiteren erfindungsgemäßen Elektrodenleitung,
- Fig. 10: eine schematische Seitenansicht eines weiteren Ausführungsbeispiel der Erfindung,
- Fig. 11: eine schematische perspektivische Darstellung eines Abschnitts einer weiteren erfindungsgemäßen Elektrodenleitung,
- Fig. 12: eine schematische Längsschnittdarstellung eines weiteren Ausführungsbeispiels der Erfindung, bei dem ein hartes Glied zusätzlich eine elektrische Funktion realisiert,
- Fig. 13: eine perspektivische Darstellung eines weiteren Ausführungsbeispieles der Erfindung,
- Fig. 14A und 14B: Seitenansichten eines Abschnitts des Elektrodenkörpers jeweils einer weiteren erfindungsgemäßen Elektrodenleitung,
- Fig. 15A und 15B: skizzenartige Darstellungen weiterer Ausführungsform der erfindungsgemäßen Elektrodenleitung und
- Fig. 16: eine skizzenartige Darstellung eines weiteren Ausführungsbeispiels.

In der nachfolgenden Beschreibung der Figuren sind jeweils für gleiche oder gleich wirkende Teile oder Abschnitte ähnliche Bezugsziffern verwendet, und vorangegangene Beschreibungen werden, soweit sie sich auf solche Teile beziehen und keine Besonderheiten gegeben sind, bei nachfolgenden Figuren nicht wiederholt.

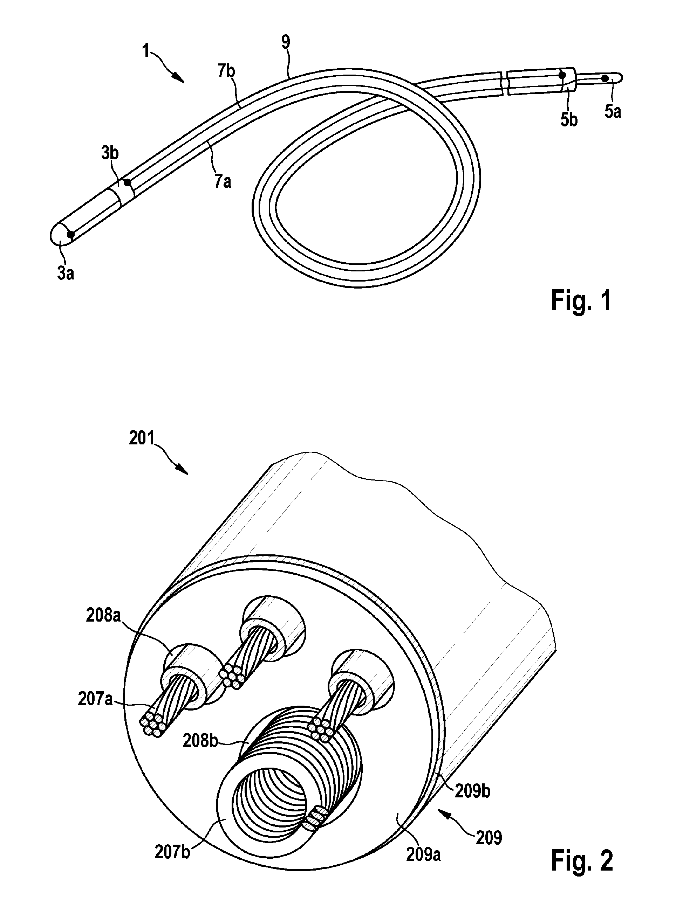

Fig. 1 zeigt schematisch eine bipolare Elektrodenleitung 1, an deren distalem Ende eine Spitzenelektrode 3a und eine Ringelektrode 3b und an deren proximalem Ende zwei entsprechende Elektrodenkontakte 5a und 5b vorgesehen sind, die mit der jeweils zugehörigen Elektrode durch eine erste und zweite Zuleitung 7a, 7b verbunden sind. Die Elektroden, Elektrodenkontakte und Zuleitungen sind auf bzw. in einem (in der Regel mehrschichtigen) Leitungskörper 9 untergebracht.

Fig. 2 zeigt in einer perspektivischen Schnittansicht mit verschiedenen Schnittebenen eine moderne Elektrodenleitung 201, bei der in einem Innenschlauch 209a - als Kern eines Zuleitungskörpers 209 - drei Lumen 208a mit kleinerem Durchmesser und ein weiteres Lumen 208b mit größerem Durchmesser vorgesehen sind. In jedem der kleineren Lumen 208a befindet sich eine Elektrodenzuleitung 207a mit Seilstruktur, die ihrerseits mit einem (nicht gesondert bezeichneten) isolierendem Mantel, z.B. aus PTFE, ETFE oder PI, versehen ist. Im größeren Lumen 208b verläuft eine Zuleitungswendel 207b, die während der Implantation zur Versteifung der Elektrodenleitung einen Führungsdraht aufnehmen kann. Zur Verbesserung der Gleit- und Abriebeigenschaften des Leitungskörpers 209 hat dieser eine Außenhülle 209b, die diese Eigenschaften positiv beeinflusst.

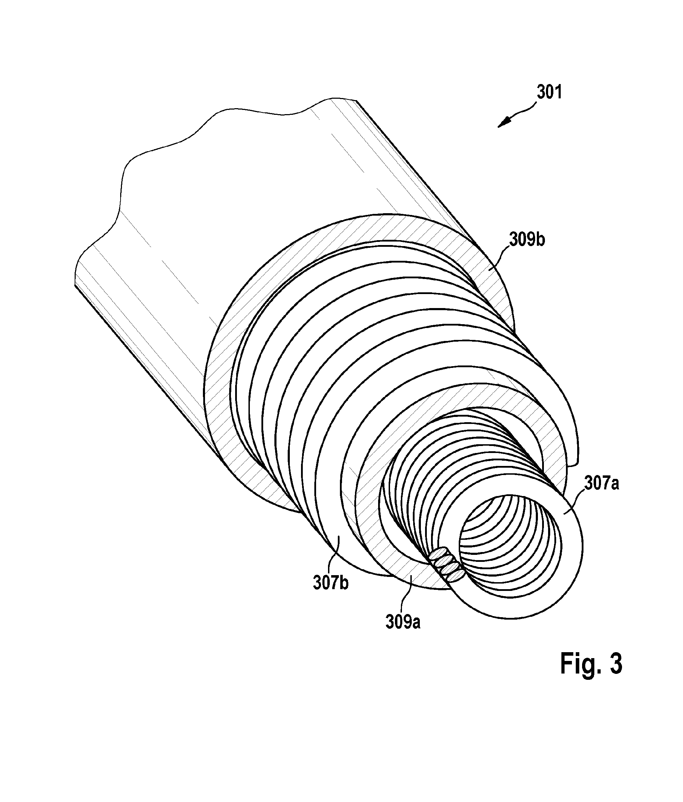

Fig. 3 zeigt eine weitere Ausführung einer implantierbaren Elektrodenleitung, bei der in koaxialer Anordnung als erste Elektrodenzuleitung (bzw. erste Gruppe von Zuleitungen) eine aus mehreren Einzeldrähten gewickelte Innenwendel 307a und koaxial hierzu eine gleichfalls aus mehreren Einzeldrähten gewickelte Außenwendel 307b angeordnet ist (die ebenfalls eine Gruppe von Elektrodenzuleitungen realisieren kann). Zwischen der Innen- und Außenwendel ist ein Silikonschlauch 309a vorgesehen, und die Außenwendel ist mit einem weiteren Isolierschlauch 309b ummantelt, der ebenfalls aus Silikon oder einem Polyurethan oder einem Copolymer bestehen kann. Auch eine Kombination aus mehreren Schläuchen ist hier einsetzbar.

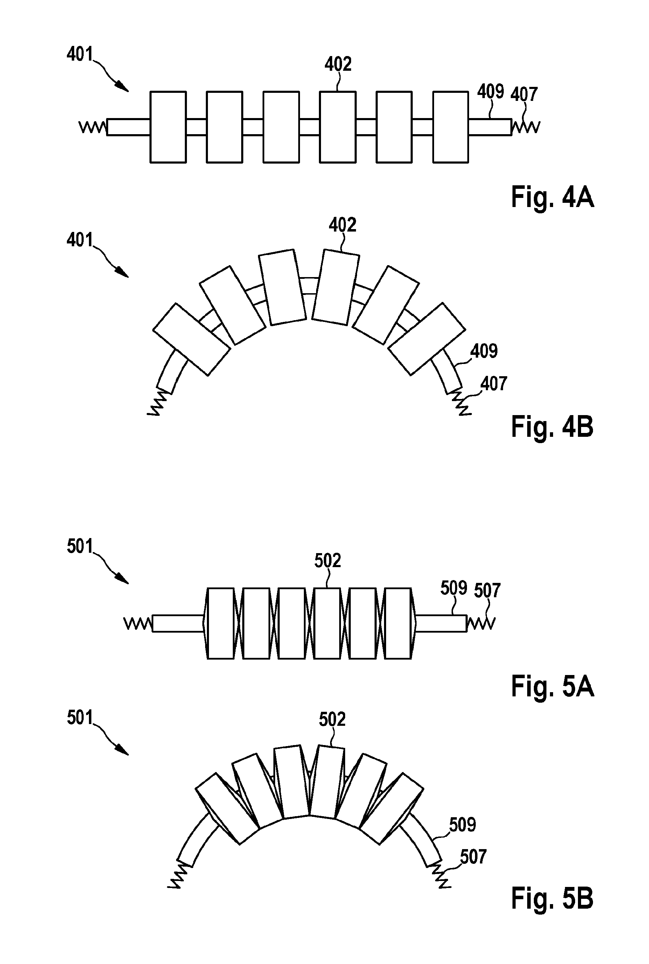

Fig. 4A und 4B zeigen schematisch in einer Seitenansicht einen erfindungsgemäß ausgestalteten Abschnitt einer Elektrodenleitung 401, in dem auf einem Leitungskörper 409, der eine Elektrodenzuleitung 407 enthält, mit kleinen Abständen zueinander eine Gruppe scheibenförmiger harter Glieder 402 als Schutz gegen starke mechanische Beanspruchung angeordnet ist. Fig. 5A und 5B zeigen eine ähnliche Elektrodenleitung 501, die sich von der vorherigen lediglich durch die dichte Reihung der schützenden harten Glieder 502 auf dem Leitungskörper 509 und die Form dieser Glieder unterscheidet. Bei diesen sind beide Stirnflächen kegelförmig (die Gesamtform also annähernd diskusförmig), was trotz dichter Reihung eine Biegung der Elektrodenleitung im besagten Abschnitt ermöglicht. Der minimale Biegeradius ist durch den Kegelwinkel der Stirnflächen der harten Glieder 502 bestimmt.

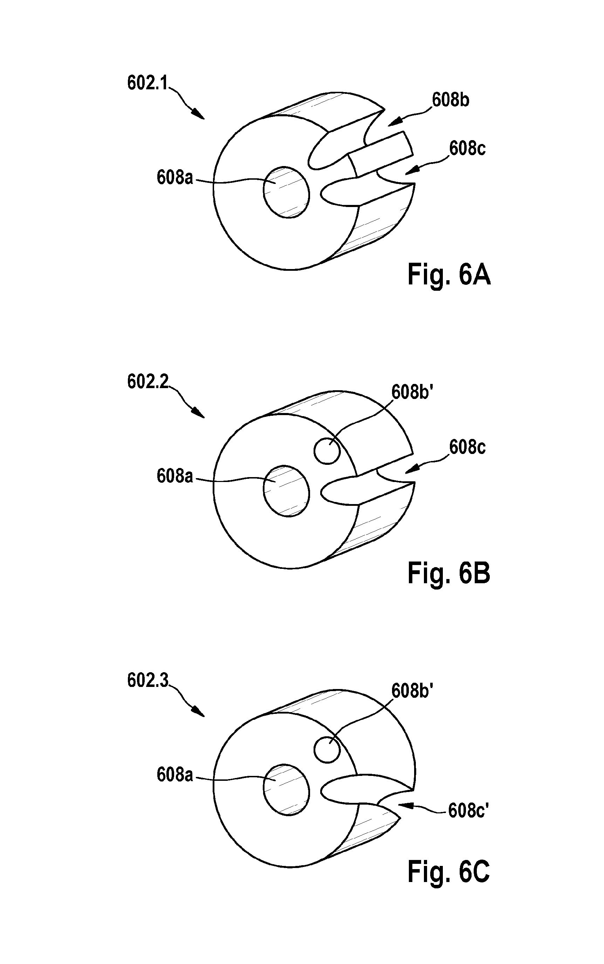

In den Figuren 6A bis 6C sind in perspektivischen Darstellungen verschieden geformte harte Glieder 602.1, 602.2 bzw. 602.3 gezeigt. Alle Ausführungen haben die Grundform eines Zylinders bzw. einer Scheibe und ein zentrales Lumen 608a für eine erste (nicht gezeigte) Elektrodenzuleitung. Das harte Glied 602.1 gemäß Fig. 6A hat überdies zwei radiale Ausnehmungen 608b und 608c, in denen weitere Elektrodenzuleitungen geführt sein können. Beim harten Glied 602.2 nach Fig. 6B und 602.3 nach Fig. 6C tritt an die Stelle der einen radial offenen Ausnehmung 608b ein zweites Innenlumen 608b'. Bei dem harten Glied 602.3 nach Fig. 6C ist überdies die verbleibende Ausnehmung 608c als Abschnitt einer Wendel gekrümmt, so dass bei Aneinanderreihung mehrerer korrespondierend geformter Glieder sich ein wendelförmiger Verlauf dieser Ausnehmung bzw. Nut ergibt, durch den ein gleichermaßen wendelförmiger Verlauf einer hierin angelegte Elektrodenzuleitung bestimmt werden kann.

Bei den in Fig. 6A bis 6C gezeigten harten Gliedern kann das zentrale Lumen 608a einen Führungsdraht, einen Schlauch, eine gewendelte Elektrodenzuleitung oder eine seilförmige Elektrodenzuleitung aufnehmen. In den äußerlich zugänglichen Ausnehmungen oder weiteren Lumen können getrennt verlaufende seilförmige oder als dünne Wendel ausgebildete Zuleitungen untergebracht werden. Im Falle der äußerlich zugänglichen Ausnehmungen können diese nachträglich auf der Elektrodenleitung angebracht werden. Bei der Unterbringung in dem kleineren Lumen 608b' entfällt zwar diese Möglichkeit, die darin verlaufende seil- oder wendelförmige Zuleitung ist jedoch besser gegenüber der Umgebung isoliert. Derart ausgebildete Glieder schützen die Zuleitung etwa, wenn sie unter einer Ringelektrode oder einer Schockwendel hindurchgeführt werden soll.

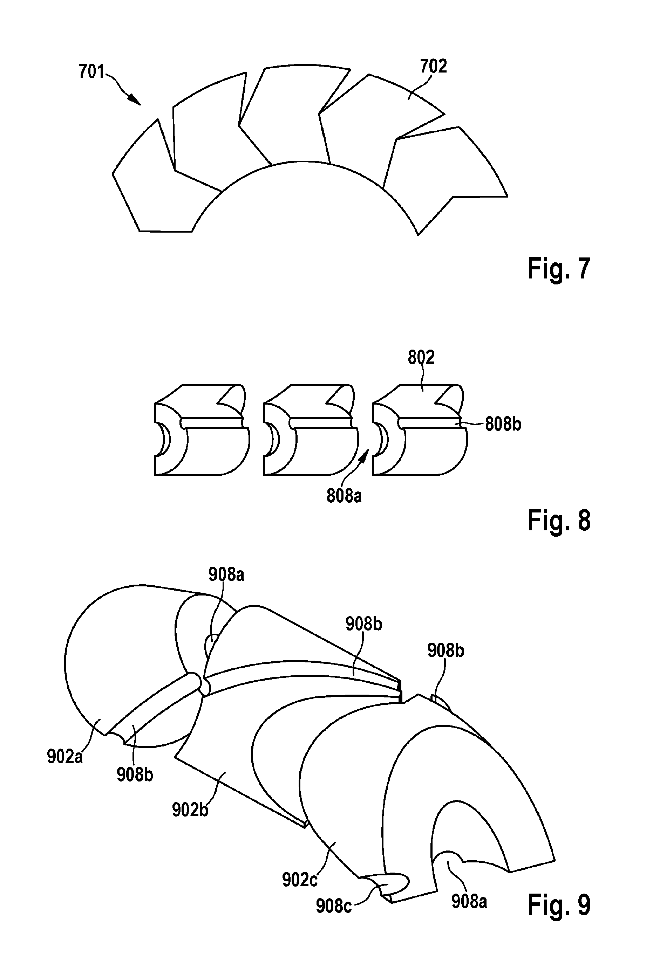

Fig. 7 zeigt in einer schematischen Seitenansicht als weiteres Ausführungsbeispiel der Erfindung eine Elektrodenleitung 701 in einem gebogenen Zustand, welche ebenfalls in einem besonderen mechanischen Beanspruchungen ausgesetzten Abschnitt aneinander gereihte harte Glieder 702 aufweist, deren Grundform zylindrisch ist und deren eine Stirnseite eine im Querschnitt dreieckige Einkerbung und deren andere Stirnseite eine in ihrer Form zu dieser Einkerbung korrespondierende vorstehende Profilierung aufweist. Ähnlich wie bei der Ausführung nach Fig. 5A und 5B ermöglicht es auch diese Formgebung der harten Glieder, dass die Elektrodenleitung mit einem vorbestimmten Mindestradius biegsam ist.

Fig. 8 zeigt als Ausgestaltung dieser Ausführungsform drei zueinander benachbarte, vom eigentlichen Leitungskörper gelöste harte Glieder 802, bei denen jeweils neben einem zentralen Lumen 808a ein seitlich versetztes kleineres Lumen 808b vorgesehen ist. Das zweite Lumen befindet sich nahe einer Symmetrieebene der harten Glieder, welche zugleich die - hierzu orthogonale - Ebene bestimmt, in der die mit solchen Gliedern versehene Elektrodenleitung biegsam ist, so dass das Seil bei einer Biegung der Elektrodenleitung weder wesentlich gestreckt noch wesentlich gestaucht wird. Hierdurch werden Relativbewegungen zwischen der in dem Lumen aufgenommenen Elektrodenzuleitung und den schützenden Gliedern weitgehend gehindert.

Eine weitere Ausgestaltung des in Fig. 7 und 8 skizzierten Konstruktionsprinzips ist in Fig. 9 in Form einer Gruppe von harten Gliedern 902a, 902b, 902c dargestellt. Neben einem zentralen Lumen 908a haben diese Glieder jeweils zwei radiale Nuten 908b und 908c, welche nicht parallel zum zentralen Lumen (und somit in Längsrichtung des jeweiligen Gliedes), sondern schräg hierzu verlaufen. Bei dieser Ausführung ist eine Gruppe von harten Gliedern in Abstimmung aufeinander derart geformt, dass die Symmetrieebene der Kerbe an einer Stirnseite orthogonal zur Orientierung der Kante an der anderen Stirnseite orientiert und zugleich ein kontinuierlicher wendelförmiger Verlauf der radialen Nuten 908b, 908c über alle Glieder der Reihung gewährleistet ist. So kann der Elektrodenkörper bei Aneinanderreihung dreier Kettenglieder bereits eine Biegung in jeder Richtung vornehmen.

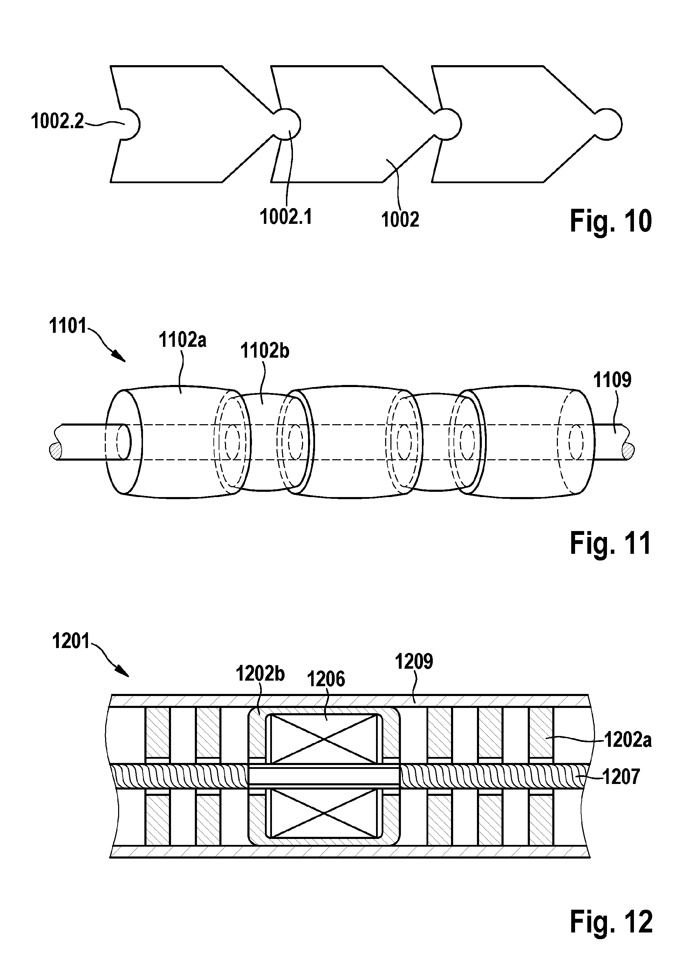

Fig. 10 zeigt schematisch als weitere Ausführung der Erfindung eine Gruppe von drei harten Gliedern 1002, die zur Anbringung auf oder in einem Elektrodenleitungskörper vorgesehen sind und sich durch das Vorsehen eines kugelartigen oder kreisscheibenförmigen Vorsprunges 1002.1 an der einen, im Übrigen kegelförmig ausgebildeten Stirnfläche und einer dazu passenden Kugelpfanne 1002.2 an der anderen, mit geringerer Steigung ebenfalls kegelförmig ausgebildeten Stirnfläche auszeichnen. Dir Kugeln bzw. Kreisscheiben und Kugel- bzw. Kreisscheibenpfannen bilden im montierten Zustand eine rotationssymmetrische Gelenkverbindung zwischen der harten Gliedern und ermöglichen damit eine allseitige Biegung einer damit ausgestalteten Elektrodenleitung.

Fig. 11 zeigt als Alternative zu der in Fig. 10 skizzierten Gelenkverbindung in Gestalt einer Elektrodenleitung 1101 eine weitere Lösung zur Gewährleistung einer hohen Biegsamkeit. Die Leitung 1101 umfasst einen Leitungskörper 1109, der üblicherweise aus einem elastischen Kunststoffmaterial besteht und dessen innere Bestandteile (speziell Zuleitungen) hier nicht dargestellt sind. Auf diesen Leitungskörper 1109 sind alternierend erste harte Glieder 1102a und zweite harte Glieder 1102b aufgeschoben. Die Darstellung ist rein schematisch, verdeutlicht aber, dass die ersten harten Glieder 1102a eine zylindrische bis fassartige Grundgestalt haben und ihre beiden Stirnflächen jeweils konkav ausgeformt sind, während die zweiten harten Glieder 1102b eine annährend sphärische Grundgestalt haben und in die konkav ausgeformten Stirnflächen der ersten harten Glieder eingreifen. Auch durch diesen Eingriff wird eine Art Kugelgelenk und somit eine Biegsamkeit der Elektrodenleitung in allen Ebenen realisiert.

Fig. 12 zeigt in einer schematischen Längschnitt-Darstellung eine weitere erfindungsgemäße Elektrodenleitung 1201, welche eine Elektrodenzuleitung 1207, eine Gruppe von ersten harten Gliedern 1202a zum Schutz der Zuleitung und einen in diesem Fall außen liegenden, die Zuleitung 1207 mit den darauf platzierten harten Gliedern 1202a ummantelnden Leitungskörper 1209 umfasst. Eine Besonderheit besteht darin, dass ein einzelnes hartes Glied 1202b eines zweiten Typs zwischen die scheibenförmigen harten Glieder 1202a eingefügt ist. Dieses andersartige Glied 1202b ist trommelförmig und enthält in seinem Inneren eine Spule 1206, welche ein zusätzliches elektrisches Bauelement darstellt und mechanisch und elektrisch mit der zentralen Elektrodenzuleitung 1207 verbunden ist. Elektrisch kann diese Verbindung als Reihenschaltung ausgelegt sein und so die Induktivität der Elektrodenzuleitung vergrößern. Das trommelförmige Gehäuse des harten Gliedes 1202b ist drehbar auf der Elektrodenzuleitung gelagert, so dass sich der Leitungskörper gegenüber der Elektrodenzuleitung mit der fest aufgesetzten Spule drehen lässt und die Entstehung von Torsionsspannungen im Gebrauch der Elektrodenleitung vermieden wird.

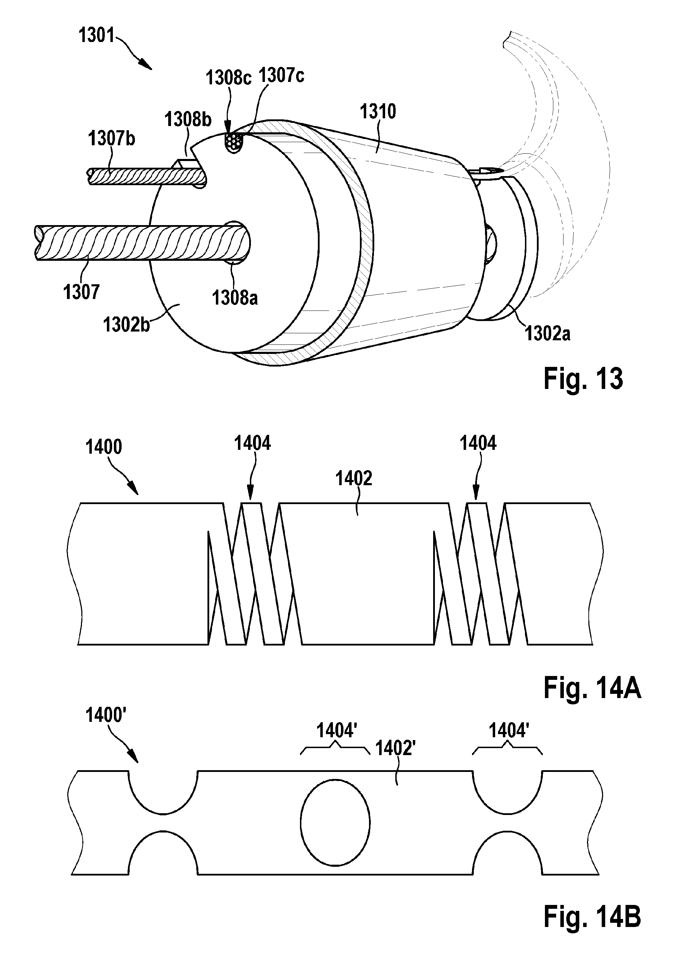

Fig. 13 zeigt als weiteres Ausführungsbeispiel der Erfindung eine Elektrodenleitung 1301, die mit harten Gliedern 1302a geschützt ist, deren Aufbau der Ausführung nach Fig. 6A ähnelt. Die Elektrodenleitung 1301 umfasst eine Ringelektrode 1310, die als Ring über dem Außenumfang eines andersartigen harten Gliedes 1302b angeordnet ist. Bei diesem Glied 1302b ist die zweite radiale Ausnehmung 1308c derart verkleinert, dass eine darin aufgenommene Zuleitung 1307c in mechanischen und elektrischen Kontakt mit der Innenwandung der Ringelektrode 1310 gezwungen und diese hierdurch elektrisch an die Zuleitung 1307c angeschlossen wird. Die Verbindung kann zusätzlich mit einem Schweißpunkt fixiert werden.

Fig. 14A und 14B zeigen zwei grundsätzlich andersartige Realisierungen von "harten Gliedern" zum Schutz einer Elektrodenleitung. In beiden Ausführungen ist ein Rohr 1400 bzw. 1400' (etwa mittels eines Laserschneidverfahrens) derart bearbeitet, dass unbearbeitete und somit starre ("harte") Abschnitte 1402 bzw. 1402' sich mit bearbeiteten und in Folge der durch die Bearbeitung geschaffenen Ausnehmungen relativ leicht verformbaren ("weichen") Abschnitten 1404 bzw. 1404' abwechseln. Die weichen Abschnitte 1404 bei der Ausführung nach Fig. 14A sind durch einen spiralig verlaufenden bandförmigen Einschnitt geschaffen. Bei der Ausführung nach Fig. 14B sind sie durch kreisförmige Einschnitte geschaffen, welche alternierend um 90° versetzt angebracht sind, so dass eine Biegsamkeit der durch das Schutzroh geschützten Elektrodenleitung in zwei Ebenen gewährleistet ist.

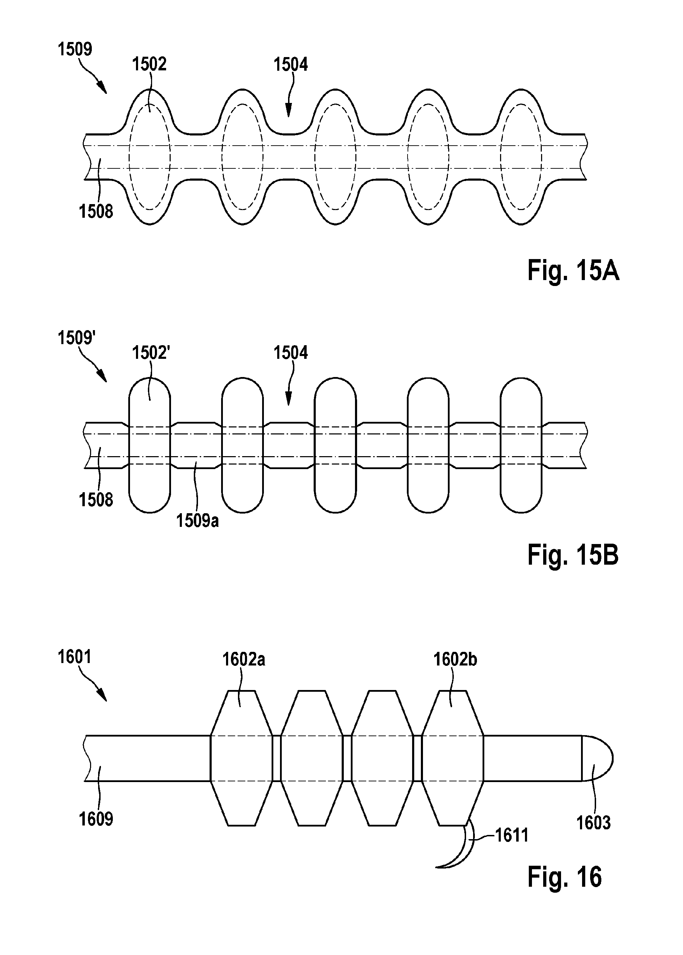

Fig. 15A zeigt zur Illustration eine weiteren Ausführung der Erfindung einen Leitungskörper 1509 mit einem zentralen Lumen 1508 zur Aufnahme von (nicht dargestellten) Elektrodenzuleitungen, welcher durch Umspritzen von mit relativ großem Abstand aufgereihten harten Gliedern 1502 mit einer elastischen Masse gebildet ist. Durch das Umspritzen mit relativ großem Abstand sind zwischen den harten Gliedern jeweils "weiche", d.h. biege- und formelastische Leitungskörperabschnitte 1504 ausgebildet, die für eine hinreichende Flexibilität der fertigen Elektrodenleitung sorgen. Fig. 15B zeigt als alternativen Aufbau mit vergleichbarer Funktion einen Leitungskörper 1509', der durch Aufpressen von abgerundet scheibenförmigen (linsenartigen) harten Gliedern 1502' auf einen Schlauch 1509a aus biegeelastischem und kompressiblem Material mit vorbestimmten Abstand zueinander gebildet ist. Auch hier sind die Abstandsbereiche 1504 zwischen den harten Gliedern 1502' relativ leicht verformbar und stellen somit eine Art gelenkige Verbindung zwischen den "harten" Abschnitten dar.

Fig. 16 zeigt skizzenartig als weitere Ausführung der Erfindung einen distalen Abschnitt einer Elektrodenleitung 1601, bei dem neben ersten, ausschließlich dem Schutz gegen mechanische Beanspruchung dienenden harten Gliedern 1602a ein Glied 1602b vorgesehen ist, welches einen nach Implantation über den Führungsdraht gesteuert ausfahrbaren (in der Figur ausgefahren dargestellten) Fixierungshaken 1611 zur Fixierung der Elektrodenleitung im Körpergewebe eines Patienten aufweist. Das Glied 1602d mit dem Fixierungshaken 1611 ist nahe einer distalen Elektrode 1603 der Leitung 1609 angeordnet.

Die Ausführung der Erfindung ist nicht auf die oben beschriebenen Beispiele und hervorgehobenen Aspekte beschränkt, sondern ebenso in einer Vielzahl von Abwandlungen möglich, die im Rahmen fachgemäßen Handelns liegen.

## Patentansprüche

1. Implantierbare Elektrodenleitung zur Übertragung elektrischer Impulse an reizbares Körpergewebe und/oder von an Körpergewebe abgegriffenen elektrischen Signalen zu einer Erfassungseinheit, mit
einer distalen Elektrode,
einem proximalen Elektrodenanschluss und
einer die oder jede Elektrode mit dem oder jedem Elektrodenanschluss verbindende, oder zur Übertragung von Elektroschocks oder zur Ansteuerung von Sensoren dienende, in einem Leitungskörper verlaufende Elektrodenzuleitung,
wobei der Leitungskörper eine gelenkige Reihung von harten Gliedern aufweist.

2. Elektrodenleitung nach Anspruch 1, wobei in die Reihung von harten Gliedern kompressible und/oder biegsame, insbesondere form- oder biegeelastische, Abschnitte eingefügt sind.

3. Elektrodenleitung nach Anspruch 1 oder 2, wobei die Reihung von harten Gliedern mindestens abschnittsweise von einem formelastischen Material umgeben oder auf einem formelastischen Material angeordnet ist.

4. Elektrodenleitung nach Anspruch 3, wobei um die Reihung von harten Gliedern ein flexibler Schlauch gezogen oder sie mit dem formelastischen Material umspritzt oder auf ein formelastisches Material aufgezogen oder aufgespritzt ist.

5. Elektrodenleitung nach einem der vorangehenden Ansprüche, wobei die harten Glieder mindestens einen der folgenden Werkstoffe aufweisen:
ein Metall, insbesondere Platin, Tantal, Iridium, Palladium, rostfreien Stahl, Gold oder MP35N,
eine Keramik, insbesondere Al2O3, ZrO2, TiO2, MgO, ZnO, Al2O3 + Ti02, BaO + Ti02, SiC, BeO, AlN, HfC, TaC, TiN, BN, B4C, WC oder Si3N4,
ein Glas,
einen Kunststoff, insbesondere PEEK, Silikon, ein Copolymeres, ein Polyimid, PA, hochdichtes Polyethylen oder Polysulfon oder faser- oder nanopartikel gefüllten Varianten der genannten Kunststoffe.

6. Elektrodenleitung nach einem der vorangehenden Ansprüche, wobei jeweils mindestens einige der harten Glieder zueinander direkt benachbart und ihre einander zugewandten Stirnflächen derart geformt sind, dass ein, insbesondere bezüglich des minimalen Biegeradius limitiertes, Verkippen um eine Längsachse der Elektrodenleitung möglich ist.

7. Elektrodenleitung nach einem der vorangehenden Ansprüche, wobei jeweils mindestens einige der harten Glieder zueinander direkt benachbart und durch Gelenkabschnitte zugfest miteinander verbunden sind.

8. Elektrodenleitung nach einem der vorangehenden Ansprüche, wobei unterschiedlich geformte und/oder verschieden große erste und zweite harte Glieder oder harte und weiche Glieder in alternierender Reihung vorgesehen sind, die gelenkartig ineinander greifen.

9. Elektrodenleitung nach einem der Ansprüche 1 bis 5, wobei die harten Glieder durch starre Abschnitte eines Rohres gebildet sind, zwischen denen biegsame Abschnitte vorliegen, die durch in die Wandung des Rohres eingearbeitete Ausnehmungen definiert sind.

10. Elektrodenleitung nach Anspruch 9, wobei die Ausnehmungen wendelförmig langgestreckt verlaufen oder als Paare oder Gruppen kreisrunder oder elliptischer Öffnungen ausgebildet sind.

11. Elektrodenleitung nach einem der vorangehenden Ansprüche, wobei mindestens eines der harten Glieder ein elektrisches oder elektronisches Bauteil, z.B. eine Elektrode, eine Spule, ein Sensorelement, einen Kondensator oder ein aktives elektronisches Bauelement oder eine elektronische Schaltung, realisiert oder, insbesondere in einem hierzu vorgesehenen Hohlraum, enthält.

12. Elektrodenleitung nach einem der vorangehenden Ansprüche, wobei mindestens eines der harten Glieder ein Fixationselement zur mechanischen Fixierung der Elektrodenleitung im implantierten Zustand aufweist.

13. Elektrodenleitung nach einem der vorangehenden Ansprüche, wobei die harten Glieder mindestens eine zentrische und/oder eine azentrische Ausnehmung zur Aufnahme der oder einer Elektrodenzuleitung aufweisen.

14. Elektrodenleitung nach Anspruch 13, wobei die oder mindestens eine Elektrodenzuleitung als, insbesondere gegenüber den harten Gliedern isolierte/s, Seil oder Wendel ausgeführt und die Ausnehmungen der harten Glieder daran angepasst sind.

15. Elektrodenleitung nach Anspruch 14, wobei eine azentrische Ausnehmung der harten Glieder so gestaltet und deren Reihung so gewählt ist, dass ein Verlauf der Elektrodenzuleitung in den sich aneinander anschließenden Ausnehmungen der zueinander benachbarten harten Glieder hierdurch vorbestimmt, insbesondere die Elektrodenzuleitung wendelförmig geführt, ist.
